# EUROPEAN PATENT APPLICATION

(11) **EP 3 106 659 A1**
(43) Date of publication of application: **21.12.2016**
(21) Application number: 15749623.3
(22) Date of filing: 13.01.2015
(51) Int. Cl.: F03D 13/00, G01N 21/59

(54) **STATE DETECTION DEVICE FOR WIND POWER GENERATION DEVICE**

(30) Priority: 12.02.2014 JP 2014024399
(71) Applicant: NTN Corporation, Osaka-shi, Osaka 550-0003 (JP)
(72) Inventor: HASEBA, Takashi, Kuwana-shi Mie 511-0811 (JP)
(74) Representative: Bockhorni & Kollegen
(86) International application number: PCT/JP2015/050615
(87) International publication number: WO 2015/122224

(57) **Abstract**

A wind power generation apparatus includes a sensor which senses a quantity of light transmitted through a lubricant supplied to a gear box internal to a nacelle, and a monitor which monitors a state of the lubricant based on an output of the sensor. The monitor calculates as a normal standard deviation σₙₒᵣₘₐₗ a standard deviation of the sensor output value in a normal operation in which a foreign matter is not introduced in the lubricant (S10), generates as a threshold value for abnormality determination σₛₕ the calculated normal standard deviation σₙₒᵣₘₐₗ multiplied by a real number (S11), and stores the generated threshold value σₛₕ (S12). And the monitor determines whether a foreign matter is introduced in the lubricant based on a result of comparing the sensor output value's standard deviation σ with threshold value σₛₕ.

## Description

### TECHNICAL FIELD

The present invention relates to a technique to detect a state of a wind power generation apparatus and particularly to a technique to detect a state of a lubricant used in the wind power generation apparatus.

### BACKGROUND ART

Japanese Patent Laying-Open No. 8-285181 (PTD 1) discloses a foreign matter detection device which detects that a foreign matter such as iron powder is introduced in a lubricant supplied to a gear box. This foreign matter detection device uses a transmission type optical fiber sensor to sense a quantity of light transmitted through a lubricant flowing through a lubricant pipe, and determines, based on a value output from the sensor, whether a foreign matter is introduced in the lubricant. And when the foreign matter detection device determines that a foreign matter is introduced in the lubricant, the foreign matter detection device turns on a lamp and issues a warning.

### CITATION LIST

### PATENT DOCUMENT

[PTD 1] Japanese Patent Laying-Open No. 8-285181

### SUMMARY OF INVENTION

### TECHNICAL PROBLEM

When a foreign matter in a lubricant is detected based on an output of a sensor which senses a quantity of light transmitted through the lubricant, as described in PTD 1, whether the foreign matter is present/absent is often determined based on a result of comparing the sensor's output value with a threshold value. Conventionally this threshold value is often determined based on the sensor's output value in a state in which a foreign matter is introduced in the lubricant. This methodology, however, requires passing a foreign matter-containing lubricant through an actual pipe in order to set the threshold value.

Furthermore, when a foreign matter-containing lubricant cannot be passed through the actual pipe, it is necessary to pass the foreign matter-containing lubricant through another pipe different from the actual pipe and set a threshold value based on a result of having measured with a sensor a quantity of light transmitted through the lubricant passing through the other pipe. However, the threshold value set by this methodology is not what is set from a quantity of light transmitted through the lubricant passing through the actual pipe, and when whether a foreign matter is present/absent is determined using the threshold value set by this methodology, there is a possibility that an erroneous determination may be made.

The present invention has been made to solve the above issue, and an object thereof is to allow whether a foreign matter is introduced in a lubricant to be determined with precision without passing a foreign matter-containing lubricant through an actual pipe.

### SOLUTION TO PROBLEM

A state detection device according to the present invention is a state detection device for a wind power generation apparatus, comprising: a sensor configured to detect a quantity of light transmitted through a lubricant for the wind power generation apparatus; and a monitor configured to monitor a state of the lubricant based on an output of the sensor. The monitor includes: a generation unit configured to calculate a normal standard deviation that is a standard deviation of output values of the sensor during a normal operation in which a foreign matter is not introduced in the lubricant, and generate a threshold value for abnormality determination by multiplying the calculated normal standard deviation by a real number; and a determination unit configured to calculate a standard deviation of output values of the sensor and determine whether the foreign matter is introduced in the lubricant based on a result of comparison between the calculated standard deviation and the threshold value generated by the generation unit.

Preferably the determination unit is configured to calculate the standard deviation of the output value of the sensor a plurality of times to obtain a plurality of standard deviations, and when a ratio of any standard deviation exceeding the threshold value to the plurality of calculated standard deviations exceeds a predetermined ratio, the determination unit is configured to determine that a foreign matter is introduced in the lubricant and issue a warning.

Preferably, the wind power generation apparatus includes a pipe allowing the lubricant to circulate therethrough. The pipe has a transparent portion formed to transmit external light. The sensor is provided outside the transparent portion of the pipe, and senses a quantity of light transmitted through the lubricant flowing through the transparent portion.

### ADVANTAGEOUS EFFECT OF INVENTION

The present invention allows whether a foreign matter is introduced in a lubricant to be determined with precision without passing a foreign matter-containing lubricant through an actual pipe.

### BRIEF DESCRIPTION OF DRAWINGS

Fig. 1 is a diagram schematically showing a configuration of a wind power generation apparatus.
Fig. 2 is a diagram schematically showing a configuration of a circulation device.
Fig. 3 is a diagram schematically showing a structure of a sensor.
Fig. 4 is a flowchart showing a flow of a process performed when a monitor performs a threshold value generating process.
Fig. 5 is a flowchart showing a flow of a process performed when the monitor performs an abnormality determination process.
Fig. 6 shows an example of a result of a calculation of a standard deviation σₙₒᵣₘₐₗ in a normal operation and a standard deviation σ in an abnormal operation.

### DESCRIPTION OF EMBODIMENTS

The present invention will now be described in an embodiment hereinafter in detail with reference to the drawings. In the figures, identical or corresponding components are identically denoted and will not be described repeatedly.

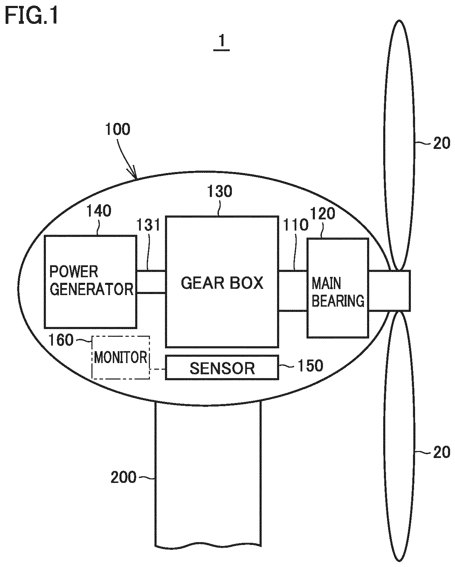

Fig. 1 is a diagram schematically showing a configuration of a wind power generation apparatus 1 including a state detection device according to the present embodiment.

Wind power generation apparatus 1 is configured including a plurality of blades 20, a nacelle 100, and a tower 200.

Nacelle 100 is provided at an upper end of tower 200. Nacelle 100 has a main shaft 110, a main bearing 120, a gear box 130, a power generator 140, a sensor 150, and a monitor 160 housed therein.

Blade 20 is provided at an end of main shaft 110, and converts wind power into a rotation torque and transmits it to main shaft 110. Main shaft 110 is rotatably supported in nacelle 100 by main bearing 120, and transmits to gear box 130 the rotation torque received from blade 20.

Gear box 130 is provided between main shaft 110 and power generator 140, accelerates the rotation speed of main shaft 110 and outputs it to power generator 140.

Power generator 140 is connected to an output shaft 131 of gear box 130 and generates electric power by the rotation torque received from gear box 130. Power generator 140 is an induction generator, for example.

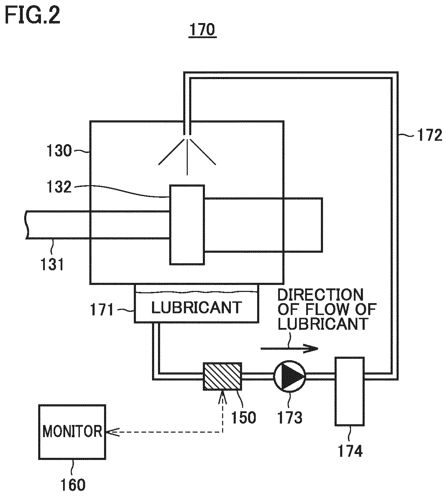

Inside nacelle 100 a circulation device 170 (see Fig. 2 described later) is provided for circulating a lubricant through gear box 130.

Fig. 2 is a diagram schematically showing a configuration of circulation device 170 for circulating a lubricant through gear box 130.

Circulation device 170 includes an oil pan 171, a pipe 172, a pump 173, and a filter 174.

Oil pan 171 is provided at a lower portion of gear box 130. Pipe 172 has one end connected to a lower portion of oil pan 171 and the other end connected to an upper portion of gear box 130. Pump 173 and filter 174 are provided on the route of pipe 172.

When pump 173 is driven, the lubricant reserved in oil pan 171 is supplied from one end of pipe 172 to filter 174 and filtered thereby. The lubricant having passed through filter 174 falls by free fall from the other end of pipe 172 provided at the upper portion of gear box 130 and is supplied to a gear 132 in gear box 130. The lubricant supplied to gear 132 is returned to oil pan 171 by free fall.

Sensor 150 is provided at a portion of pipe 172 between oil pan 171 and pump 173. Note that the position of sensor 150 is not necessarily limited to a portion of pipe 172 between oil pan 171 and pump 173, as long as it is on the route of pipe 172.

Monitor 160 is composed of a unit having a CPU (a central processing unit) and a memory (not shown) incorporated therein and determines, based on information stored in the memory and an output value of sensor 150, etc., whether a foreign matter such as iron powder is introduced in a lubricant passing through pipe 172. When monitor 160 determines that a foreign matter is introduced in the lubricant, it issues a warning by turning on a lamp (not shown) etc.

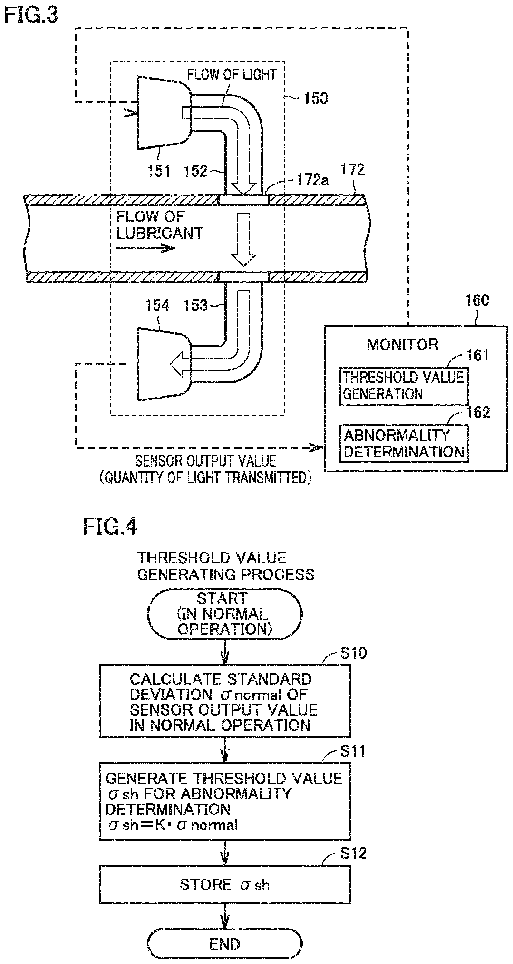

Fig. 3 is a diagram schematically showing a structure of sensor 150.

Sensor 150 is a transmission type optical fiber sensor which senses a quantity of light transmitted through a lubricant supplied to gear box 130. Sensor 150 includes a light emitting device 151, optical fibers 152, 153, and a light receiving device 154.

Pipe 172 has a portion provided with a transparent portion 172a formed of a transparent material such as acrylic resin.

Optical fibers 152, 153 are disposed outside transparent portion 172a of pipe 172, spaced as prescribed, and face each other.

Light emitting device 151 generates light in response to a control signal issued from monitor 160. The light generated by light emitting device 151 passes through optical fiber 152 and is sent to the lubricant flowing through transparent portion 172a of pipe 172. The lubricant transmits the light, which is guided to light receiving device 154 by optical fiber 153. A quantity of transmitted light received by light receiving device 154 is sent to monitor 160 as an output value of sensor 150 (hereinafter also referred to as a "sensor output value").

The quantity of transmitted light received by light receiving device 154 (or a sensor output value) decreases more when a foreign matter (e.g., iron powder) is introduced in larger amounts in the lubricant flowing through transparent portion 172a. By this effect, the sensor output value has a larger variation when an amount of a foreign matter introduced in the lubricant is equal to or greater than a prescribed amount (hereinafter also referred to as "in an abnormal operation") than when the amount of the foreign matter introduced in the lubricant is less than the prescribed amount (hereinafter also referred to as "in a normal operation").

Focusing on this point, monitor 160 according to the present embodiment calculates a standard deviation σₙₒᵣₘₐₗ of the sensor output value in the normal operation (hereinafter also referred to as a "normal standard deviation"), and uses the calculated normal standard deviation σₙₒᵣₘₐₗ to statistically generate a threshold value for abnormality determination σₛₕ.

More specifically, monitor 160 includes a threshold value generation unit 161 and an abnormality determination unit 162, as shown in Fig. 3. Threshold value generation unit 161 calculates normal standard deviation σₙₒᵣₘₐₗ and calculates threshold value σₛₕ by multiplying the calculated normal standard deviation σₙₒᵣₘₐₗ by a real number (hereinafter this process is also referred to as a "threshold value generating process"). Abnormality determination unit 162 calculates the sensor output value's standard deviation σ, and determines whether abnormality is present/absent based on a result of comparing the calculated standard deviation σ with threshold value σₛₕ (hereinafter this process is also referred to as an "abnormality determination process").

Fig. 4 is a flowchart showing a flow of a process performed when monitor 160 (more specifically, threshold value generation unit 161 shown in Fig. 3) performs the threshold value generating process. This flowchart is started in the normal operation (for example immediately after the lubricant is exchanged etc.).

In S10, monitor 160 calculates the sensor output value's standard deviation in the normal operation as normal standard deviation σₙₒᵣₘₐₗ. More specifically, monitor 160 samples the sensor output value in the normal operation for each prescribed period of time, and calculates a standard deviation of a plurality of thus sampled pieces of data as normal standard deviation σₙₒᵣₘₐₗ. Note that the standard deviation can be calculated in a known methodology.

In S 11, monitor 160 calculates as threshold value for abnormality determination σₛₕ the calculated normal standard deviation σₙₒᵣₘₐₗ multiplied by a coefficient K. Herein, coefficient K is a real number predetermined based on an experiment etc., and in the present embodiment it is set to "3".

In S12, monitor 160 stores the calculated threshold value σₛₕ to the memory. Note that when a previously calculated threshold value σₛₕ is stored in the memory, monitor 160 deletes the previously calculated threshold value σₛₕ and updates threshold value σₛₕ by a currently calculated, new threshold value σₛₕ. Note that, for an initial value (a value when the threshold value generating process is not yet performed) of threshold value σₛₕ, for example a value previously obtained for example via an experiment etc. can be stored.

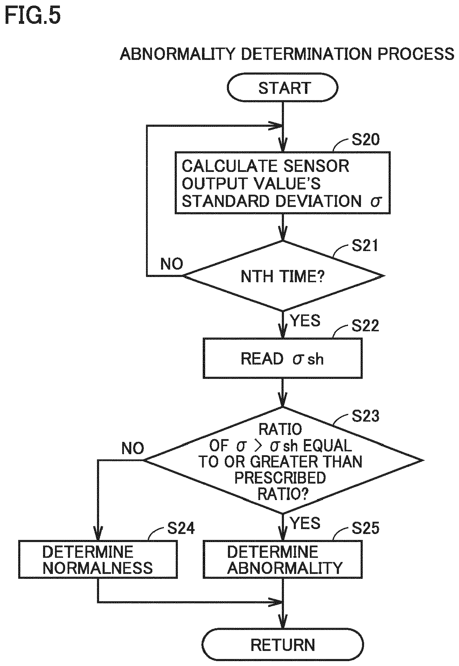

Fig. 5 is a flowchart showing a flow of a process performed when monitor 160 (more specifically, abnormality determination unit 162 shown in Fig. 3) performs the abnormality determination process. This flowchart is repeatedly performed periodically as prescribed.

In S20, monitor 160 calculates the sensor output value's standard deviation σ. Note that, as well as the methodology used to calculate normal standard deviation σₙₒᵣₘₐₗ, as described above, the methodology used to calculate standard deviation σ may per se be a known methodology.

In S21, monitor 160 determines whether standard deviation σ is calculated n times. Herein, "n" is a predetermined natural number equal to or greater than two (e.g., "5").

When standard deviation σ is calculated less than n times (NO in S21), i.e., when the number of calculated standard deviations σ is less than n, monitor 160 returns the processing to S20, and calculates a next standard deviation σ. For example, when data for 10 seconds is used to calculate standard deviation σ once, the calculation of standard deviation σ will be performed every 10 seconds and this is done n times.

When standard deviation σ is calculated n times (YES in S21), i.e., when n standard deviations σ are calculated, monitor 160 in S22 reads from the memory threshold value σₛₕ (= K · σₙₒᵣₘₐₗ) generated by the above described threshold value generating process.

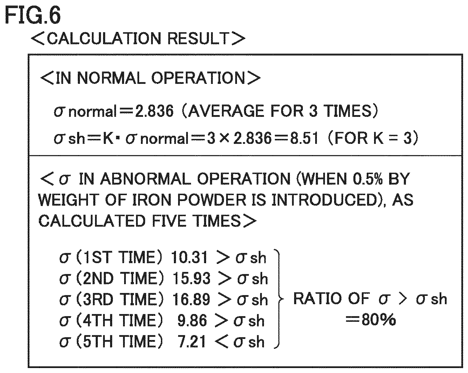

Then monitor 160 determines in S23 whether a ratio of standard deviation σ exceeding threshold value σₛₕ to n standard deviations σ is equal to or greater than a prescribed ratio. Herein, the prescribed ratio is a value (e.g., about 80%) previously set by an experiment etc. (see Fig. 6 described hereinafter).

When the ratio exceeding threshold value σₛₕ is less than the prescribed ratio (NO at S23), monitor 160 determines normalness (i.e., that the amount of a foreign matter introduced in the lubricant is less than a prescribed amount) at S24.

In contrast, when the ratio exceeding threshold value σₛₕ is equal to or greater than the prescribed ratio (YES at S23), then monitor 160 in S25 determines abnormality (i.e., that the amount of a foreign matter introduced in the lubricant is greater than or equal to the prescribed amount), and issues a warning.

Fig. 6 shows an example of a result of a calculation of standard deviation σₙₒᵣₘₐₗ in the normal operation and standard deviation σ in the abnormal operation.

In the example shown in Fig. 6, standard deviation σₙₒᵣₘₐₗ in the normal operation is "2.836." Note that this value is an average of values obtained from calculating standard deviation σₙₒᵣₘₐₗ in the normal operation 3 times. When the above coefficient K is set to "3" threshold value for abnormality determination σₛₕ will be "8.51" (= 3 x 2.836).

In contrast, a result of a calculation of standard deviation σ in the abnormal operation (for the Fig. 6 example, when 0.5% by weight of iron powder is introduced into the lubricant) five times is "10.31," "15.93," "16.89," "9.86," and "7.21."

Accordingly, in this exemplary calculation result, the ratio of the standard deviation σ that exceeds threshold value σₛₕ (= 3 x σₙₒᵣₘₐₗ) to the five standard deviations σ obtained in the abnormal operation will be 80%. Accordingly, by setting the "prescribed ratio" used in the step of S23 in Fig. 5 to about 80%, it can be determined that 0.5% by weight of iron powder is introduced in the lubricant.

Thus, the state detection device according to the present embodiment includes sensor 150 which senses a quantity of light transmitted through a lubricant used for wind power generation apparatus 1, and monitor 160 which monitors a state of the lubricant based on an output of sensor 150. Monitor 160 calculates as normal standard deviation σₙₒᵣₘₐₗ a standard deviation of a sensor output value in a normal operation in which a foreign matter is not introduced in the lubricant, and monitor 160 generates as threshold value for abnormality determination σₛₕ the calculated normal standard deviation σₙₒᵣₘₐₗ multiplied by a real number (K). Thus threshold value for abnormality determination σₛₕ can be automatically generated without passing a foreign matter-containing lubricant through actual pipe 172. This can reduce the number of steps required to generate threshold value σₛₕ, as compared with a case in which a foreign matter-containing lubricant is passed through actual pipe 172.

And monitor 160 determines whether a foreign matter is introduced in the lubricant based on a result of comparing the sensor output value's standard deviation σ with threshold value σₛₕ Thus whether a foreign matter is introduced in the lubricant can be determined without passing a foreign matter-containing lubricant through actual pipe 172. Furthermore, as threshold value σₛₕ is a value generated using actual pipe 172, whether a foreign matter is introduced in the lubricant can be determined more precisely than for example when a threshold value is generated using another pipe which is different from actual pipe 172.

In particular, according to the present embodiment, monitor 160 calculates the sensor output value's standard deviation σ n times, and when a ratio of standard deviation σ exceeding threshold value σₛₕ to the n calculated standard deviations σ exceeds a prescribed ratio (e.g., 80%), monitor 160 determines that a foreign matter is introduced in the lubricant. When a foreign matter is not introduced in the lubricant and despite that standard deviation σ exceeds threshold value σₛₕ due to noise or the like for example only once, an erroneous determination that a foreign matter is introduced in the lubricant can nonetheless be prevented as appropriate. This allows more reliable abnormality determination.

Furthermore, in the present embodiment, pipe 172 passing the lubricant has a portion provided with transparent portion 172a formed to be capable of transmitting external light. Sensor 150 is provided outside transparent portion 172a of pipe 172, and senses a quantity of light transmitted through the lubricant flowing through transparent portion 172a. This allows the lubricant's flow resistance to be suppressed and the sensor to be installed relatively easily, as compared with a case in which a portion of the sensor is provided inside pipe 172.

It should be understood that the embodiment disclosed herein have been described for the purpose of illustration only and in a non-restrictive manner in any respect. The scope of the present invention is defined by the terms of the claims, rather than the embodiment described above, and is intended to include any modifications within the meaning and scope equivalent to the terms of the claims.

### REFERENCE SIGNS LIST

1: wind power generation apparatus; 20: blade; 100: nacelle; 110: main shaft; 120: main bearing; 130: gear box; 131: output shaft; 132: gear; 140: power generator; 150: sensor; 151: light emitting device; 152, 153: optical fiber; 154: light receiving device; 160: monitor; 161: threshold value generation unit; 162: abnormality determination unit; 170: circulation device; 171: oil pan; 172: pipe; 172a: transparent portion; 173: pump; 174: filter; 200: tower.

## Claims

1. A state detection device for a wind power generation apparatus, comprising:
a sensor configured to detect a quantity of light transmitted through a lubricant for the wind power generation apparatus; and
a monitor configured to monitor a state of the lubricant based on an output of the sensor;
the monitor including:
a generation unit configured to calculate a normal standard deviation that is a standard deviation of output values of the sensor during a normal operation in which a foreign matter is not introduced in the lubricant, and generate a threshold value for abnormality determination by multiplying the calculated normal standard deviation by a real number; and
a determination unit configured to calculate a standard deviation of output values of the sensor and determine whether the foreign matter is introduced in the lubricant based on a result of comparison between the calculated standard deviation and the threshold value generated by the generation unit.

2. The state detection device for a wind power generation apparatus according to claim 1, wherein the determination unit is configured to calculate the standard deviation of the output values of the sensor a plurality of times to obtain a plurality of standard deviations, and
when a ratio of any standard deviation exceeding the threshold value to the plurality of calculated standard deviations exceeds a predetermined ratio, the determination unit is configured to determine that the foreign matter is introduced in the lubricant and issue a warning.

3. The state detection device for a wind power generation apparatus according to claim 1 or 2, wherein:
the wind power generation apparatus includes a pipe allowing the lubricant to circulate therethrough;
the pipe has a transparent portion formed to transmit external light therethrough; and
the sensor is provided outside the transparent portion of the pipe and senses a quantity of light transmitted through the lubricant flowing through the transparent portion.
